# EUROPEAN PATENT SPECIFICATION

(11) **EP 1 673 107 B1**
(45) Date of publication and mention of the grant of the patent: **26.03.2008**
(21) Application number: 04766880.1
(22) Date of filing: 07.10.2004
(51) Int. Cl.: A61K 45/06, A61K 31/506, A61K 31/4178, A61P 9/14

(54) **PHARMACEUTICAL COMPOSITION COMPRISING A SELECTIVE I1 IMIDAZOLINE RECEPTOR AGONIST AND AN ANGIOTENSIN II RECEPTOR BLOCKER**
PHARMAZEUTISCHE ZUSAMMENSETZUNG MIT EINEM SELEKTIVEN I1 IMIDAZOLIN-REZEPTOR-AGONISTEN UND EINEM ANGIOTENSIN-II-REZEPTORBLOCKER
COMPOSITIONS CONTENANT UN AGONISTE SELECTIF DES RECEPTEURS DE L'IMIDAZOLINE ET UN ANTAGONISTE DES RECEPTEURS A L'ANGIOTENSINE II

(30) Priority: 10.10.2003 EP 03103763
(43) Date of publication of application: 28.06.2006
(73) Proprietor: Solvay Pharmaceuticals GmbH, 30173 Hannover (DE)
(72) Inventor: BAUM, Dominique, B. Desai Road (Breach Candy) Mumbai 400026 (IN); BIELENBERG, Gerhard-Wilhelm, 31061 Alfeld (DE); BOEDECKER, Bernd, 30171 Hannover (DE); THORMAEHLEN, Dirk, 30952 Ronnenberg (DE)
(74) Representative: Bauriegel, Lutz
(86) International application number: PCT/EP2004/052468
(87) International publication number: WO 2005/039639

(56) References cited:
- FARSANG C: "MOXONIDINE: CLINICAL PROFILE" JOURNAL OF CLINICAL AND BASIC CARDIOLOGY, XX, AU, vol. 4, no. 3, 2001, pages 197-200, XP008026181 ISSN: 1561-2775 cited in the application
- GOODMAN ET AL: "THE PHARMACOLOGICAL BASIS OF THERAPEUTICS" PHARMACOLOGICAL BASIS OF THERAPEUTICS, GOODMAN GILMAN'S PHARMACOLOGICAL BASIS OF THERAPEUTICS, NEW YORK, PERGAMON PRESS, US, vol. ED. 10, 2001, pages 894-895, XP002268126
- ARANDA P.: "Irbesartan plus moxonidine: a "logical combination for hypertensive patients not responding to monotherapy (CONFERECE ABSTRACT: 13TH SCIENTIFIC MEETING OF THE INTERNAMERICAN SOCIETY OF HYPERTENSION" HYPERTENSION, vol. 33, no. 4, 1999, page 0165, XP008027300 cited in the application
- VETTER H ET AL: "MODERNE BASIS- UND KOMBINATIONSTHERAPIE DER HYPERTONIE MODERN BASIC AND COMBINATION THERAPY OF HYPERTENSION" NIEREN- UND HOCHDRUCKKRANKHEITEN, XX, XX, vol. 26, no. 3, 1997, pages 105-107, XP008026969 cited in the application
- SCHACHTER M ET AL: "ANTIHYPERTENSIVE EFFICACY OF MOXONIDINE IN PRIMARY CARE: A 'REAL-LIFE' STUDY" INTERNATIONAL JOURNAL OF CLINICAL PRACTICE, MEDICON INTERNATIONAL, ESHER, GB, vol. 57, no. 6, July 2003 (2003-07), pages 479-482, XP008026173 ISSN: 1368-5031
- REID J L: "NEW THERAPEUTIC AGENTS FOR HYPERTENSION" BRITISH JOURNAL OF CLINICAL PHARMACOLOGY, BLACKWELL SCIENTIFIC PUBL, GB, vol. 42, no. 1, 1996, pages 37-41, XP008026172 ISSN: 0306-5251
- TRENKWALDER P: "Combination therapy with AT1-receptor blockers" JOURNAL OF HUMAN HYPERTENSION 2002 UNITED KINGDOM, vol. 16, no. SUPPL. 3, 2002, pages S17-S25, XP008027302 ISSN: 0950-9240 cited in the application
- WETHMAR U ET AL: "INTERACTIONS OF LIGANDS AT ANGIOTENSIN II-RECEPTORS AND IMIDAZOLINE RECEPTORS" JAPANESE JOURNAL OF PHARMACOLOGY, THE JAPANESE PHARMACOLOGICAL SOCIETY, KYOTO, JP, vol. 85, no. 2, 2001, pages 167-174, XP008026171 ISSN: 0021-5198

## Description

### FIELD OF INVENTION

The present invention relates to pharmaceutical compositions comprising Moxonidine and Eprosartan mesylate. The invention also relates to the use of said compositions in the manufacture of a medicament for the treatment of hypertension, especially in hypertensive patients already suffering from type II diabetes or being susceptible to developing type II diabetes.

### BACKGROUND OF THE INVENTION

The publications and other materials used herein illuminate the background of the invention, and in particular, cases to provide additional details respecting the practice.

Recent studies such as the HOT (Hypertension Optimal Treatment) study have demonstrated the benefits of reducing blood pressure to below previously existing target levels [Hansson L. et al. (1998) Lancet 351 (9118):1755-62]. As a result of the HOT and other trials the target blood pressure levels recommended by hypertension management guidelines have become increasingly stringent during recent years. In 2003, official hypertension guidelines recommend even more effective blood pressure lowering. The new ESC/ESH guidelines recommend to reduce blood pressure in all hypertensive patients to at least below 140/90 mm Hg, and below 130/80 mmHg in diabetics [European Society of Hypertension-European Society of Cardiology Guidelines Committee (2003) J Hypertens. 21(6):1011-53; and Chobanian AV et al. (2003) JAMA. 289(19):2560-72]. A trend towards even lower target levels can be expected in the future.

Although current hypertension management guidelines recommend increasingly stringent blood pressure targets, these targets are seldom achieved in clinical practice by one single drug.

In particular, systolic blood pressure is generally poorly controlled [Taylor Nelson Sofres Healthcare, Cardio Monitor® Study (1998)]. Even in patients with mild to moderate hypertension, mono-therapy is only effective in approximately 50 - 70 % of the patients and thus there is a clear need for combination therapy if stringent blood pressure targets are to be achieved. Drugs used in combination therapy should satisfy a number of prerequisites, including complementary mechanism of action, enhanced efficacy in combination and maintained (or improved) tolerability [Trenkwalder P. (2002) J of Human Hypertension 16, Suppl 3: S17- S25].

The sympathetic nervous system (SNS) and the renin-angiotensin-aldosterone system (RAAS) are both contributors to the development and maintenance of hypertension [Rupp H & Jäger B. (2001) J Clin Basic Cardiol 4:47-51]. Activation of the SNS results in increased vasomotor tone and is thus causally related to the development and maintenance of high blood pressure. The RAAS on the other hand plays an important role in the physiological regulation of cardiovascular, renal and endocrine functions. Overactivation of this system contributes to the development and persistence of various forms of hypertension.

Within the TOPIC study, it was already demonstrated that the combined therapy with Moxonidine and the Angiotensin Converting Enzyme (ACE)-Inhibitor Enalapril shows a positive effect. The combination was effective in 27 % of those hypertensive patients being refractory to monotherapy with Moxonidine [Waters J. et al. (1999) J Clin Bas Cardiol. 2(2):219-24; Prichard et al. (2002) Blood Press 11(3):166-72].

A further review article by Vetter and Düsing suggests the combination of Moxonidine with ACE inhibitors as an example of useful combinations. Furthermore, the quadruple combination of a diuretic, a calcium channel blocker (CCB), Moxonidine and an ACE inhibitor or an angiotensin II receptor (AT1) blockers (ARB) is mentioned [Vetter H & Düsing R. (1997) Nieren- und Hochdruckkrankheiten 26(31):105-107].

In addition, within a further document it was suggested to combine Moxonidine with other antihypertensives, for example with ACE inhibitors and ARBs. A reduction of the central sympathetic tone through Imidazoline I1 receptor activation by Moxonidine in combination with the inhibition of the RAAS by an ARB may produce additive/synergistic antihypertensive effects [Farsang C. (2001) J Clin Basic Cardiol 4:197-200].

Furthermore, Aranda et al. disclosed the synergistic antihypertensive effects of a combination therapy with Irbesartan and Moxonidine in patients with moderate essential hypertension who were unresponsive to monotherapy [Aranda P. et al. (1999) (Conference abstract: 13th Scientific Meeting of the Inter-American Society of Hypertension, USA) Hypertension. 33(4):1065]
However, there is a clear need for novel and effective approaches in combination therapy in order to achieve a stringent control of the blood pressure levels, particularly with regard to the newly established target levels according to the recently proposed European and US guidelines [see above]. Especially patients with type II diabetes require two or more medications in order to reduce their blood pressure to the proposed low levels- [Zanchetti A & Ruilope LM (2002) Hypertension; 20:2099-2110]. These target blood pressure levels are 130/80 mm Hg in diabetic subjects with proteinuria of up to 1 g/day and 125/75 mm Hg in those with proteinuria in excess of 1 g/day. Compliance issues surrounding the prescribing of several drug classes (coupled with spiraling costs for poly-pharmacy) will fuel the continued increase and acceptance of fixed dose combination products. Drugs used in combination therapy should satisfy a number of prerequisites, including complementary mechanism of action, enhanced efficacy in combination and maintained (or improved) tolerability.

### SUMMARY OF THE INVENTION

Therefore, it is an object of the present invention to develop novel pharmaceutical compositions for the effective treatment of hypertension by the combination of drugs with different mechanisms of action in order to achieve a stringent control of blood pressure target levels, especially for the (pre)-diabetic hypertensive patient.

It now has been found, that the combined administration of the Imidazoline I1 receptor agonist Moxonidine and the angiotensin (AT1) receptor blocker (ARB) Eprosartan fulfills these criteria due to the complementary pharmacological properties of both drugs. The combined Moxonidine and Eprosartan therapy is well suited for the treatment of hypertension, particularly systolic hypertension and hypertension associated with metabolic and renal impairment and heart failure, because this drug combination inhibits the two main pressure systems SNS (sympathetic nervous system) and RAAS (renin-angiotensin-aldosterone system) and consequently inhibits neuro-hormonal activation.

The combination of two completely different modes of actions provides a powerful alter native to current Hydrochlorothiazide (HCTZ) combinations as well as offering the possibility of having greater protection for diabetic and renally impaired patients.

Accordingly, the present invention relates to a pharmaceutical composition comprising Moxonidine as a selective I1 imidazoline receptor agonist or a pharmaceutically acceptable salt thereof and Eprosartan as an angiotensin II receptor blocker (ARB) or a pharmaceutically acceptable salt thereof and a pharmaceutically acceptable carrier.

In particular, the present invention relates to a pharmaceutical composition consisting of a fixed combination of moxonidine and eprosartan mesylate. In a further preferred embodiment of the invention, the pharmaceutical composition comprises 0.05 - 1 mg, preferably 0.2 - 0.6 mg, of Moxonidine, and 900 - 1000 mg, preferably 200 - 800 mg, more preferably 300 - 600 mg, of Eprosartan, corresponding to 122.6 - 1226.3 mg, preferably 245.2 mg - 980.8 mg, more preferably 367.9 - 735.8 mg, of Eprosartan mesylate. In particular, the present invention relates to a pharmaceutical composition, wherein the Moxonidine is present in a dose of 0.2 mg, 0.3 mg, 0.4 mg or 0.6 mg and the Eprosartan is present in a dose of 400 mg, 600 mg or 800 mg. Most preferred, the present invention relates to a pharmaceutical composition, wherein the Moxonidine is present in a dose of 0.2 mg or 0.4 mg and the Eprosartan is present in a dose of 600 mg.

The pharmaceutical composition according to the present invention can be in the form of a tablet consisting mainly of Eprosartan and further of Moxonidine homogenously distributed within the Eprosartan. Alternatively, the pharmaceutical composition can be in the form of a coated tablet wherein a small Moxonidine containing core is coated with an Eprosartan containing blend. Additionally, the present invention relates to a pharmaceutical composition in the form of an Eprosartan containing tablet core coated with a thin layer comprising the Moxonidine. Furthermore, the pharmaceutical composition can be in the form of a bilayer tablet or in the form of a trilayer tablet. All mentioned types of tablets may be provided with an additional coating, e.g. in order to impart taste masking and / or a specific drug release profile.

In a further embodiment according to the present invention, the pharmaceutical composition additionally comprises a diuretic, in particular hydrochlorothiazide.

Furthermore, the present invention relates to the novel use of a therapeutically effective amount of Moxonidine as selective I1 imidazoline receptor agonist and of a therapeutically effective amount of Eprosartan as angiotensin II receptor blocker for the manufacture of a medicament for the treatment of a subject suffering from or being susceptible to hypertension, in particular systolic hypertension.
In a preferred embodiment of the invention, a therapeutically effective amount of Moxonidine as selective I1 imidazoline receptor agonist and of a therapeutically effective amount of Eprosartan is used for the manufacture of a medicament for the treatment of a subject suffering from or being susceptible to hypertension, in particular systolic hypertension. Preferably, the Eprosartan is administered in a daily dosage range from 100 - 1000 mg, preferably 200 - 800 mg, most preferably 300 - 600 mg.

In a further preferred embodiment of the present invention, a therapeutically effective amount of Moxonidine and a therapeutically effective amount of Eprosartan as angiotensin II receptor blocker are used for the manufacture of a medicament for the treatment of a subject suffering from or being susceptible to hypertension, in particular systolic hypertension. Preferably, the Moxonidine is administered in a daily dosage range from 0.05 - 1 mg, preferably from 0.2 - 0.6 mg.

Additionally, it is an object of the present invention to use Eprosartan in a daily dosage of 400 mg, 600 mg or 800 mg and Moxonidine in a daily dosage of 0.2 mg, 0.3 mg, 0.4 mg or 0.6 mg for the manufacture of a medicament for the treatment of a subject suffering from or being susceptible to hypertension, in particular systolic hypertension. Preferably, it is an object of the present invention to use Eprosartan in a daily dosage of 600 mg and Moxonidine in a daily dosage of 0.2 or 0.4 mg for the manufacture of a medicament for the treatment of a subject suffering from or being susceptible to hypertension, in particular systolic hypertension.

A preferred embodiment of the present invention relates to the use of any of the above indicated combinations for the manufacture of a medicament for the treatment of a subject suffering from or being susceptible to hypertension, in particular systolic hypertension, associated with metabolic impairment. In particular, the metabolic impairment is characterized by insulin resistance, hyperglycemia, diabetes mellitus type II, and/or hyperlipidemia. Additionally, the subject can suffer from or be susceptible to hypertension, in particular systolic hypertension, associated with diabetes mellitus type II. Furthermore, the hypertension can be associated with renal impairment and/or heart failure.

Furthermore, the present invention relates to the novel use of a therapeutically effective amount of Moxonidine as selective I1 imidazoline receptor agonist, of a therapeutically effective amount of Eprosartan as angiotensin II receptor blocker and additionally of a therapeutically effective amount of a diuretic, in particular hydrochlorothiazide, for the manufacture of a medicament for the treatment of a subject suffering from or being susceptible to hypertension, in particular systolic hypertension, and related diseases as defined above.

### DETAILED DESCRIPTION OF THE INVENTION

### I. DEFINITIONS AND NOMENCLATURE

Before describing the present invention in detail, it is to be understood that this invention is not limited to specific dosage forms, carriers, or the like, as such may vary. It is also to be understood that the terminology used herein is for the purpose of describing particular embodiments only, and is not intended to be limiting.

It must be noted that as used in this specification and the appended claims, the singular forms "a," "an", and "the" include plural referents unless the context clearly dictates otherwise. Thus, for example, reference to "an active agent" or "a pharmacologically active agent" includes a single active agent as well as two or more different active agents in combination, reference to "a carrier" includes mixtures of two or more carriers as well as a single carrier, and the like.

In describing and claiming the present invention, the following terminology will be used in accordance with the definitions set out below.

The terms "active agent," "pharmacologically active agent" and "drug" are used interchangeably herein to refer to a chemical compound that induces a desired pharmacological, physiological effect. The primary active agents herein are inhibitors of the renin-angiotensin system, in particular **Eprosartan** as angiotensin II receptor antagonists, and **Moxonidine** as selective imidazoline receptor agonists. The terms also encompass pharmaceutically acceptable, pharmacologically active derivatives of those active agents specifically mentioned herein, including, but not limited to, salts, esters, amides, prodrugs, active metabolites, analogs, and the like. When the terms "active agent," "pharmacologically active agent" and "drug" are used, then, or when an active agent such as an angiotensin II receptor antagonist or a selective imidazoline receptor agonist is specifically identified, it is to be understood that applicants intend to include the active agent per se as well as pharmaceutically acceptable, pharmacologically active salts.

The term "selective imidazoline receptor agonist" as used herein refers to a pharmacologically active, pharmaceutically acceptable agent that binds selectively to the 11 subtype of imidazoline receptor (I1R). The selective imidazoline receptor agonists represent a new class of centrally acting antihypertensive agents that have been developed to control blood pressure effectively without the adverse effects of sedation and mental depression that usually are associated with centrally acting antihypertensive agents. This new generation of centrally acting antihypertensive agents is selective for the imidazoline receptor but has a low affinity for alpha(2)-adrenergic receptors.

The term "inhibitor of the renin-angiotensin system" as used herein refers to a pharmacologically active, pharmaceutically acceptable agent that inhibits, directly or indirectly, the adverse effects of angiotensin, particularly angiotensin II. Included, without limitation, are agents that: inhibit angiotensin II synthesis; inhibit angiotensin II binding to the AT₁ receptor; or inhibit renin activity.

The terms "angiotensin II receptor antagonist" or "angiotensin II receptor blockers" as used herein refer to a pharmacologically active, pharmaceutically acceptable agent that block the angiotensin II Type 1 (AT(1)) receptor by inhibiting angiotensin II binding to the AT₁ receptor without effecting other hormone systems.

The term "diuretic" as used herein refers to a pharmacologically active, pharmaceutically acceptable agent that can be used in the treatment of hypertension and management of edema, such as with congestive heart failure.

By "pharmaceutically acceptable," such as in the recitation of a "pharmaceutically acceptable carrier," or a "pharmaceutically acceptable acid addition salt," is meant herein a material that is not biologically or otherwise undesirable, i.e., the material may be incorporated into a pharmaceutical composition administered to a patient without causing any undesirable biological effects or interacting in a deleterious manner with any of the other components of the composition in which it is contained. "Pharmacologically active" (or simply "active"), as in a "pharmacologically active" derivative or metabolite, refers to a derivative or metabolite having the same type of pharmacological activity as the parent compound and approximately equivalent in degree. When the term "pharmaceutically acceptable" is used to refer to a derivative (e.g., a salt) of an active agent, it is to be understood that the compound is pharmacologically active as well, i.e., therapeutically effective to reduce elevated blood pressure.

"Carriers" or "vehicles" as used herein refer to conventional pharmaceutically acceptable excipient materials suitable for drug administration, and include any such materials known in the art that are nontoxic and do not interact with other components of a pharmaceutical composition or drug delivery system in a deleterious manner.

By an "effective" amount or a "therapeutically effective amount" of a drug or pharmacologically active agent is meant a nontoxic but sufficient amount of the drug or agent to provide the desired effect. In the combination therapy of the present invention, an "effective amount" of one component of the combination is the amount of that compound that is effective to provide the desired effect when used in combination with the other components of the combination. The amount that is "effective" will vary from subject to subject, depending on the age and general condition of the individual, the particular active agent or agents, and the like. Thus, it is not always possible to specify an exact "effective amount." However, an appropriate "effective" amount in any individual case may be determined by one of ordinary skill in the art using routine experimentation.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. Thus, for example, "treating" a patient involves prevention of a particular disorder or adverse physiological event in a susceptible individual as well as treatment of a clinically symptomatic individual.

### II. THE ACTIVE AGENTS

### Selective 11 imidazoline receptor agonists

The I1 subtype of imidazoline receptors (I1R) is a plasma membrane protein that is involved in diverse physiological functions. The 11-imidazoline receptor is a novel neurotransmitter receptor found mainly in the brainstem, adrenal medulla and kidney. The receptor functions at the cellular level works through arachidonic acid and phospholipid signaling cascades in neuronal cells with the net result of inhibiting sympathetic premotor neurons. The imidazoline receptors have been discovered to be involved in the central nervous system control of sympathetic outflow. A new class of centrally acting antihypertensive agents, the imidazoline receptor agonists have been developed to control blood pressure effectively without the adverse effects of sedation and mental depression that usually are associated with centrally acting antihypertensive agents. This new generation of centrally acting antihypertensive agents is highly selective for the imidazoline receptor but has a low affinity for alpha(2)-adrenergic receptors.

In particular, the 5-[(2-Imidazolin-2-yl)-amino]-pyrimidine derivatives disclosed within German patent application DE 28 49 537, which possess blood pressure lowering properties, belong to the group of selective 11 imidazoline receptor agonists. The present invention relates to the use of the compound 4-Chloro-5-[(4,5-dihydro-1H-imidazol-2-yl)-amino]-6-methoxy-2-methylpyrimidine (= Moxonidine) of the formula I

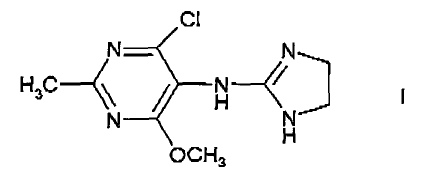

Pharmaceutical compositions comprising Moxonidine are e.g. available under the trade name Physiotens®, Cynt®, Moxon and are used as antihypertensives. It is well known in the state of the art, that moxonidine represents a selective ligand of the I1 subtype of imidazoline receptors (I1R) [Ernsberger (2000) J Cardiovasc Pharmacol, 35:S27-41]. Said compounds can be prepared according to the well-known procedures described in the aforementioned patent applications or similarly to these procedures. The anti-hyperglycemic properties of Moxonidine are well known [EP 0689837]. Furthermore, Moxonidine is able to reduce plasma insulin already in patients with impaired glucose tolerance where fasting plasma glucose is not yet influenced.

### Angiotensin receptor blockers

Angiotensin II (AII) is a potent vasoconstrictor. Its generation in the renin-angiotensin cascade results from the enzymatic action of renin on a blood plasma α2-globulin, angiotensinogen, to produce angiotensin I (AI). AI is then converted by angiotensin converting enzyme (ACE) to the octapeptide hormone All. Angiotensin II binds to angiotensin subtype I (AT₁) and subtype 2 (AT₂) receptors, as well as to several other receptors. All the known physiological effects of angiotensin II are apparently due to its binding to, and activation of, the AT₁ receptor, which is abundantly expressed in the tissues affected by angiotensin II. Angiotensin II has been implicated as a causative agent in hypertension. inhibiting the renin-angiotensin-aldosterone system (RAAS) through the use of angiotensin-converting enzyme (ACE) inhibitors which inhibit the production of All via inhibition of the angiotensin converting enzyme has proven very useful in the treatment of hypertension, congestive heart failure (CHF) and progressive renal failure. More recently, agents that directly block the angiotensin II Type 1 (AT(1)) receptor- so called "angiotensin II receptor antagonists or blockers" (AIIRAs or ARBs) - have been developed. Most of these nonpeptide angiotensin II receptor antagonists are directed at the AT₁ receptor. Angiotensin II receptor antagonists are generally highly specific, having very little effect on other hormone receptors as do the ACE inhibitors or on ion channels. Whether such specificity results in a different efficacy profile is still being determined. However, these drugs are extremely well-tolerated and very safe. ARBs are effective in the reduction of both systolic and diastolic blood pressure and compare favorably to other classes of agents. ARBs are effective in slowing the progression of renal failure in patients with Type II diabetes and may be effective in other proteinuric conditions. Overall, ARBs represent an important addition to the armamentarium of cardiovascular therapies with an excellent safety record and an emerging profile of utility in multiple cardiovascular conditions [Shusterman N. (2002) Expert Opin Drug Saf. 1(2):137-52].

the angiotensin II receptor antagonist is eprosartan.

Eprosartan mesylate (Eprosartan) is a new imidazolyl-alkenoic acids disclosed within European patent EP 0 403 159 B1 and US Pat No. 5,185,351, Eprosartan, a compound of the formula II 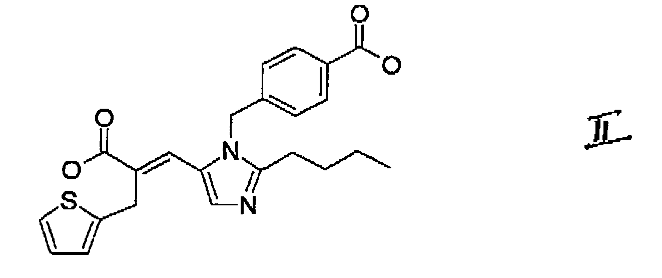 is a well known angiotension II receptor antagonist and is suited for the treatment of hypertension, congestive heart failure and renal failure.

### Diuretics

The "diuretic" employed in a composition of the present invention may be any suitable diuretic, or combination of two or more diuretics, such as acetazolamide, amiloride, azosemide, bendroflumethiazide, benzothiazide, bumetanide, chlorothiazide, chlorthalidone, clopamide, cyclopenthiazide, cyclothiazide, dichlorphenamide, dorzolamide, ethacrynate sodium, ethacrynic acid, ethoxzolamide, furosemide, hydrochlorothiazide, hydroflumethiazide, indapamide, mefruside, methazolamide, methylclothiazide, metolazone, metozalone, muzolimide, piretanide, polythiazide, quinethazone, spironolactone, thrichlormethiazide, torsemide, triamterene, trichlormethiazide, tripamide, xipamide. Preferably, the diuretic is hydrochlorothiazide (6-chloro-3,4-dihydro-2H-1 ,2,4-benzothiadiazine-7-sulphonamide-1 ,1 -dioxide).

### Derivatives

Any of the active agents may be administered in the form of a salt, provided that the salt is pharmaceutically acceptable and pharmacologically active in the present context. Salts of the active agents may be prepared using standard procedures known to those skilled in the art of synthetic organic chemistry and described, for example, by J. March, Advanced Organic Chemistry: Reactions, Mechanisms and Structure, 4th Edition (New York: Wiley-Interscience, 1992).

For example, acid addition salts are prepared from a drug in the form of a free base using conventional methodology involving reaction of the free base with an acid. Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt may be reconverted to the free base by treatment with a suitable base. Conversely, preparation of basic salts of acid moieties that may be present on an active agent may be carried out in a similar manner using a pharmaceutically acceptable base such as sodium hydroxide, potassium hydroxide, ammonium hydroxide, calcium hydroxide, trimethylamine, or the like.

### III. PHARMACEUTICAL COMPOSITIONS AND DOSAGE FORMS

Oral dosage forms are used to administer the combination of active agents, and include tablets, capsules, caplets, solutions, suspensions, and/or syrups, and may also comprise a plurality of granules, beads, powders, or pellets that may or may not be encapsulated. Such dosage forms are prepared using conventional methods known to those in the field of pharmaceutical formulation and described in the pertinent texts, e.g., in Gennaro, A. R. (ed.), Remington: The Science and Practice of Pharmacy, 20th Edition (Lippincott, Williams and Wilkins, 2000). Tablets and capsules represent the most convenient oral dosage forms, in which cases solid pharmaceutical carriers are employed.

Tablets may be manufactured using standard tablet processing procedures and equipment. One method for forming tablets is by direct compression of a powdered, crystalline, or granular composition containing the active agent(s), alone or in combination with one or more carriers, additives, or the like. As an alternative to direct compression, tablets can be prepared using wet-granulation or dry-granulation processes. Tablets may also be molded rather than compressed, starting with a moist or otherwise tractable material; however, compression and granulation techniques are preferred.

In addition to the active agent(s), then, tablets prepared for oral administration using the method of the invention will generally contain other materials such as binders, diluents, lubricants, disintegrants, fillers, stabilizers, surfactants, coloring agents, and the like. Binders are used to impart cohesive qualities to a tablet, and thus ensure that the tablet remains intact after compression. Suitable binder materials include, but are not limited to, starch (including corn starch and pregelatinized starch), gelatin, sugars (including sucrose, glucose, dextrose and lactose), polyethylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, ethyl cellulose, hydroxyethyl cellulose, and the like), and Veegum. Diluents are typically necessary to increase bulk so that a practical size tablet is ultimately provided. Suitable diluents include dicalcium phosphate, calcium sulfate, lactose, cellulose, kaolin, mannitol, sodium chloride, dry starch, and powdered sugar. Lubricants are used to facilitate tablet manufacture; examples of suitable lubricants include, for example, magnesium stearate, calcium stearate, and stearic acid. Disintegrants are used to facilitate disintegration of the tablet, and are generally starches, clays, celluloses, algins, gums, or crosslinked polymers. Fillers include, for example, materials such as silicon dioxide, titanium dioxide, alumina, talc, kaolin, powdered cellulose, and microcrystalline cellulose, as well as soluble materials such as mannitol, urea, sucrose, lactose, dextrose, sodium chloride, and sorbitol. Stabilizers are used to inhibit or retard drug decomposition reactions that include, by way of example, oxidative reactions. Surfactants may be anionic, cationic, amphoteric, or nonionic surface active agents.

The dosage form may also be a capsule, in which case the active agent-containing composition may be encapsulated in the form of a liquid or solid (including particulates such as granules, beads, powders, or pellets). Suitable capsules may be either hard or soft, and are generally made of gelatin, starch, or a cellulosic material, with gelatin capsules preferred. Two-piece hard gelatin capsules are preferably sealed, such as with gelatin bands or the like. See, for example, Remington: The Science and Practice of Pharmacy, cited supra, which describes materials and methods for preparing encapsulated pharmaceuticals. If the active agent-containing composition is present within the capsule in liquid form, a liquid carrier is necessary to dissolve the active agent(s). The carrier must be compatible with the capsule material and all components of the pharmaceutical composition, and must be suitable for ingestion.

When two or more active agents are combined in a single pharmaceutical dosage form, possible interactions among the active agents, and among the active agents and the excipients, must be considered. Such consideration is well within the purview of those skilled in the art of pharmaceutical formulation. For example, eprosartan mesylate is acidic and may react with basic compounds or alkali esters in such a way as to cause hydrolysis and/or degradation of other compounds, e.g. moxonidine. The present composition thus encompasses pharmaceutical compositions wherein two or more of the active agents are separated from each other within the pharmaceutical dosage form, by, for example, separating potentially interacting compounds from each other within the pharmaceutical dosage form, as in separate flat layers of a tablet (e.g., a bilayer or trilayer tablet), concentric or other coat-type layers, coated beads or granules (which may be incorporated into a compressed tablet or into a capsule), and/or by using buffers (see, for example, U.S. Pat. No. 6,235,311). It will also be appreciated by those in the art that such dosage forms, wherein two or more active agents are physically separated from the other active agents, can be manufactured so that different active agents will have different release profiles, e.g., if one active agent is formulated with an enteric coating, another active agent is formulated in a sustained release matrix, and the like. Alternatively, non-reactive pharmaceutically active derivatives of one or more of the potentially interacting compounds may be used.

Solid dosage forms, whether tablets, capsules, caplets, or particulates, may, if desired, be coated so as to provide for taste masking and / or delayed release. Dosage forms with delayed release coatings may be manufactured using standard coating procedures and equipment. Such procedures are known to those skilled in the art and described in the pertinent texts, e.g., in Remington, supra. Generally, after preparation of the solid dosage form, a delayed release coating composition is applied using a coating pan, an airless spray technique, fluidized bed coating equipment, or the like. Delayed release coating compositions comprise a polymeric material, e.g., cellulose butyrate phthalate, cellulose hydrogen phthalate, cellulose proprionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, polymers and copolymers formed from acrylic acid, methacrylic acid, and/or esters thereof.

Sustained release dosage forms provide for drug release over an extended time period, and may or may not be delayed release. Generally, as will be appreciated by those of ordinary skill in the art, sustained release dosage forms are formulated by dispersing a drug within a matrix of a gradually bioerodible (hydrolyzable) material such as an insoluble plastic, a hydrophilic polymer, or a fatty compound, or by coating a solid, drug-containing dosage form with such a material. Insoluble plastic matrices may be comprised of, for example, polyvinyl chloride or polyethylene. Hydrophilic polymers useful for providing a sustained release coating or matrix cellulosic polymers include, without limitation: cellulosic polymers such as hydroxypropyl cellulose, hydroxyethyl cellulose, hydroxypropyl methyl cellulose, methyl cellulose, ethyl cellulose, cellulose acetate, cellulose acetate phthalate, cellulose acetate trimellitate, hydroxypropylmethyl cellulose phthalate, hydroxypropylcellulose phthalate, cellulose hexahydrophthalate, cellulose acetate hexahydrophthalate, and carboxymethylcellulose sodium; acrylic acid polymers and copolymers, preferably formed from acrylic acid, methacrylic acid, acrylic acid alkyl esters, methacrylic acid alkyl esters, and the like, e.g. copolymers of acrylic acid, methacrylic acid, methyl acrylate, ethyl acrylate, methyl methacrylate and/or ethyl methacrylate, with a terpolymer of ethyl acrylate, methyl methacrylate, and trimethylammonioethyl methacrylate chloride (sold under the tradename Eudragit RS) preferred; vinyl polymers and copolymers such as polyvinyl pyrrolidone, polyvinyl acetate, polyvinylacetate phthalate, vinylacetate crotonic acid copolymer, and ethylene-vinyl acetate copolymers; zein; and shellac, ammoniated shellac, shellac-acetyl alcohol, and shellac n-butyl stearate. Fatty compounds for use as a sustained release matrix material include, but are not limited to, waxes generally (e.g., carnauba wax) and glyceryl tristearate.

### IV. UTILITY AND ADMINISTRATION

The compositions of this invention are suited for the treatment of individuals who are suffering from or being susceptible to hypertension, in particular systolic hypertension, or hypertension associated with metabolic impairment (insulin resistance, hyperglycemia, diabetes mellitus type II, and/or hyperlipidemia) and/or renal impairment and/or heart failure. In particular, the compositions of this invention are suited for the treatment of individuals who are suffering from hypertension associated diabetes mellitus type and/or at (pre)-diabetic hypertensive patients which need a stringent control of their blood pressure levels.

On average, diabetic individuals are twice as likely to have hypertension as non-diabetics. Recent recommendations point towards a tight blood pressure control in hypertensive diabetics, following the UK Prospective Diabetes Study (UKPDS). Tight control of blood pressure in type 2 diabetic patients results in a clinically important reduction in the risk of deaths related to diabetes, complications related to diabetes, progression of diabetic retinopathy and deterioration in visual activity. Type 2 Diabetes most often occurs in overweight or obese adults after the age of 30 and is often preceded by insulin resistance and/or hyperglycemia, which is also related to coronary heart disease. Factors that contribute to insulin resistance and type 2 Diabetes include genetics, obesity, physical inactivity and advancing age, all of which are also major predisposing risks for hypertension and cardiovascular disease. The relationship, therefore, between diabetes, hypertension and microvascular and macrovascular complications is complex.

There is a clear need for novel and effective approaches in combination therapy in order to achieve a stringent control of the blood pressure levels to below previously existing target levels. Especially patients with type II diabetes require two or more medications in order to reduce their blood pressure to the proposed low levels. These target blood pressure levels are 130/80 mm Hg in diabetic subjects with proteinuria of up to 1 g/day and 125/75 mm Hg in those with proteinuria in excess of 1 g/day. Many (pre-)diabetic hypertensive individuals who are in a clear need of a stringent control of their blood pressure are not optimally treated for this condition, commonly due to the lack of an effective, safe, and convenient therapy. As therapy would be chronic for (pre)-diabetic hypertensive patients, probably for the life of the patient, it should be simple and convenient for the patient. A high compliance rate for chronic therapy is found when a drug is administered orally once per day.

In a preferred embodiment of the present invention, the combination of **Moxonidine** as selective imidazoline receptor agonist and **Eprosartan** as angiotensin II receptor blocker and optionally the diuretic is comprised within a single unit-dose tablet or capsule for once-daily dosing. The present invention thus addresses a major medical need by providing an effective, safe, simple, and convenient way to lower the blood pressure level in hypertensive patients, especially in (pre-) diabetic patients, which has a high probability for patient compliance.

It is strongly preferred that the active agents be administered in a single dosage form, as emphasized above. However, in some cases, a patient may be given each active agent in its own separate dosage form, or a combination of individual "combination" dosage forms containing two or more of the present active agents. When separate dosage forms are used, Moxonidine as the selective imidazoline receptor agonist and Eprosartan as the angiotensin II receptor blocker and optionally the diuretic can be administered at essentially the same time (concurrently), or at separately staggered times (sequentially). Optimum beneficial effects are achieved when the active blood level concentrations of each active agent are maintained at substantially the same time, meaning that simultaneous drug administration is generally preferred. A single oral dosage form comprising all the active agents is, however, much preferred. Such a dosage form provides convenience and simplicity for the patient, thus increasing the chances for patient compliance, especially in patients who already take multiple medications due to existing heart disease or other diseases.

Since two or even three active agents are being used together in a combination therapy, the potency of each of the agents and the interactive effects achieved by combining them together must also be taken into account. A consideration of these factors is well within the purview of the ordinarily skilled clinician for the purpose of determining the therapeutically effective or prophylactically effective dosage amounts.

Preferred oral dosage forms contain a therapeutically effective unit dose of each active agent, wherein the unit dose is suitable for once-daily oral administration. The therapeutically effective unit dose of any particular active agent will depend, of course, on the active agent, the needs of the patient, and on other factors known to the prescribing physician. Those of ordinary skill in the art of pharmaceutical formulation can readily deduce suitable unit doses for various active agents. In general, however, the therapeutically effective unit dosages for each of the active agents are as follows:

Angiotensin II receptor blocker: approximately 1 mg to approximately 1000 mg of Eprosartan angiotensin II receptor blocker. Preferably, 100 - 1000 mg, more preferably 200 - 800 mg, most preferably 300 - 600 mg of Eprosartan.

Selective imidazoline receptor agonist: approximately 0.05 mg to approximately 20 mg of **Moxodinine** as selective imidazoline receptor agonist. Preferably, 0.1 - 0.6 mg, more preferably 0.2 - 0.4 mg of Moxonidine.

Diuretic: optionally, approximately 1 mg to approximately 500 mg of the diuretic, preferably 5 - 50 mg of hydrochlorothiazide.

In a particularly preferred embodiment, the active ingredients are as follows:
600 mg of Eprosartan
0.2 mg of Moxonidine

In a particularly preferred embodiment, the active ingredients are as follows:
600 mg of Eprosartan
0.3 mg of Moxonidine

In a particularly preferred embodiment, the active ingredients are as follows:
600 mg of Eprosartan
0.4 mg of Moxonidine

In a particularly preferred embodiment, the active ingredients are as follows:
400 mg of Eprosartan
0.2 mg of Moxonidine

In a particularly preferred embodiment, the active ingredients are as follows:
400 mg of Eprosartan
0.3 mg of Moxonidine

In a particularly preferred embodiment, the active ingredients are as follows:
400 mg of Eprosartan
0.4 mg of Moxonidine

In a particularly preferred embodiment, the active ingredients are as follows:
600 mg of Eprosartan
0.4 mg of Moxonidine
12.5 mg of Hydrochlorothiazide

In a particularly preferred embodiment, the active ingredients are as follows:
600 mg of Eprosartan
0.4 mg of Moxonidine
25 mg of Hydrochlorothiazide

The formulations of the invention will be administered for as long as the patient suffers from or is susceptible to hypertension, in particular systolic hypertension, or hypertension associated with metabolic impairment (insulin resistance, hyperglycemia, diabetes mellitus type II, and/or hyperlipidemia) and/or renal impairment and/or heart failure; very likely, this will be for a prolonged period and possibly for the life of the patient. Administration for at least one to two weeks is required for minimal benefit to be achieved. In addition to the preferred formulations designed for daily dosing, sustained release forms of such formulations may be employed, which may provide for dosing biweekly, weekly, monthly, or the like.

### V. PACKAGED KITS

In another embodiment, a packaged kit is provided that contains a plurality of oral dosage forms for self administration; a container means, preferably sealed, for housing the dosage forms during storage and prior to use; and instructions for a patient to carry out drug administration. The instructions will typically be written instructions on a package insert, a label, and/or on other components of the kit, and the oral dosage forms are as described herein. Each dosage form may be individually housed, as in a sheet of a metal foil-plastic laminate with each dosage form isolated from the others in individual cells or bubbles, or the dosage forms may be housed in a single container, as in a plastic bottle. The present kits will also typically include means for packaging the individual kit components, i.e., the dosage forms, the container means, and the written instructions for use. Such packaging means may take the form of a cardboard or paper box, a plastic or foil pouch, etc.

### FIGURES

Figure 1: Effect of oral treatment with moxonidine (Mox, 1 mg/kg), eprosartan (Epro, 3 mg/kg) or their combination on the systolic blood pressure of renal hypertensive rats 6 weeks after narrowing of the right renal artery.
Figure 2: Effect of oral treatment with moxonidine (Mox, 1 mg/kg), eprosartan (Epro, 3 mg/kg) or their combination on the diastolic blood pressure of renal hypertensive rats 6 weeks after narrowing of the right renal artery.

### EXPERIMENTAL

The practice of the present invention will employ, unless otherwise indicated, conventional techniques of pharmaceutical formulation and the like, which are within the skill of the art. Such techniques are fully explained in the literature. In the following examples, efforts have been made to ensure accuracy with respect to numbers used (e.g., amounts, temperatures, etc.) but some experimental error and deviation should be accounted for. Unless otherwise indicated, temperature is in degrees Celsius and pressure is at or near atmospheric pressure at sea level. All reagents were obtained commercially unless otherwise indicated.

### EXAMPLE 1

A tablet formulation was produced by a high shear Fielder granulation. The purified water is added during granulation to form the dihydrate of the Eprosartan salt. The film coat is applied to a level of approximately 2.5 - 4 % of core weight.

| Ingredients | Amounts |
|---|---|
| | (& w/w) |
| Intragranular | |
| Eprosartan mesylate (400 mg as zwitterion) | 61.32 |
| Lactose, Monohydrate (Impalpable) NF | 3.59 |
| Microcrystalline Cellulose (Avicel PH102) NF | 3.59 |
| Pregelatinized starch (Starch 1551) USP | 3.59 |
| Purified water USP | 4.36 |
| | |
| Extragranular | |
| Croscarmellose sodium (Ac-Di-Sol) | 4.00 |
| Microcrystalline cellulose (Avicel PH102) NF | 18.74 |
| Moxonidine (0.4 mg) | 0.06 |
| Magnesium stearate | 0.75 |
| Tablet core weight | 100 |
| | |
| Film coating: Opadry Blue OY-S-20900 | |

### EXAMPLE 2 - PHARMACOLOGICAL ASSAY FOR HYPERTENSION

### 1. Introduction

The effect of a combined administration of moxonidine, as an example for an selective imidazoline I1-receptor agonist, and eprosartan, as an example for an angiotensin II AT₁ receptor antagonist, was analysed by measuring their influence on the blood pressure and heart rate of 2K1 C (two-kidney one-clip) hypertensive rats. The "two-kidney one-clip" technique results in renal ischemia and in the development of hypertension. In the rat, this technique produces chronic changes, similar to those in human beings with unilateral renal artery stenosis. 2K1 C rats represent a pressure overload model of hypertension, characterized by the activation of the renin-angiotensin aldosterone system (RAAS) and peripheral vasoconstriction. This model is widely used as a high renin model of hypertension for the evaluation of angiotensin converting enzyme (ACE) inhibitors and angiotensin receptor antagonists (ARBs).

### 2. Methods

Animals: Male Sprague-Dawley CFY rats were used. Animals were fed commercial laboratory rat food pellet and allowed to drink tap water ad libitum throughout the experiments.

Two-kidney one-clip hypertension: Under ether anesthesia an incision was made on the right side of the dorsalvertebrocostal angle. After light exterioration of the right kidney a silver clip was placed on the renal artery, close to its origin from the aorta. The left kidney was not disturbed.

Measurement of blood pressure and heart rate: Blood pressure and heart rate were measured by the tail cuff method (Model 229, IITC Inc., CA, USA) from the 1st week after clipping the renal artery, once a week for 6 weeks. At the time of blood pressure measurements the animals were minimally warmed to an ambient temperature of 29 °C using a thermostated warming chamber.

Experimental protocol: Animals developing stable hypertension during the 6-week-study period were used for the drug treatments. 12 animals with established hypertension were used for each treatment group as follows:
- Vehicle
- Moxonidine 1 mg/kg
- Eprosartan 3 mg/kg
- Moxonidine 1 mg/kg + Eprosartan 3 mg/kg

The animals were treated orally and the blood pressure was measured 30 min, 1 h, 2 h and 4 hours there after. Different treatments were applied randomly during the day.

Statistical analysis: Parameters were expressed as mean ± standard error of the mean (SE) and after analysis of variance were compared by means of the modified 't'-statistical method of Wallenstein et al. [Wallenstein S et al. (1980) Circ. Res. 47:1-9].

### 3. Results

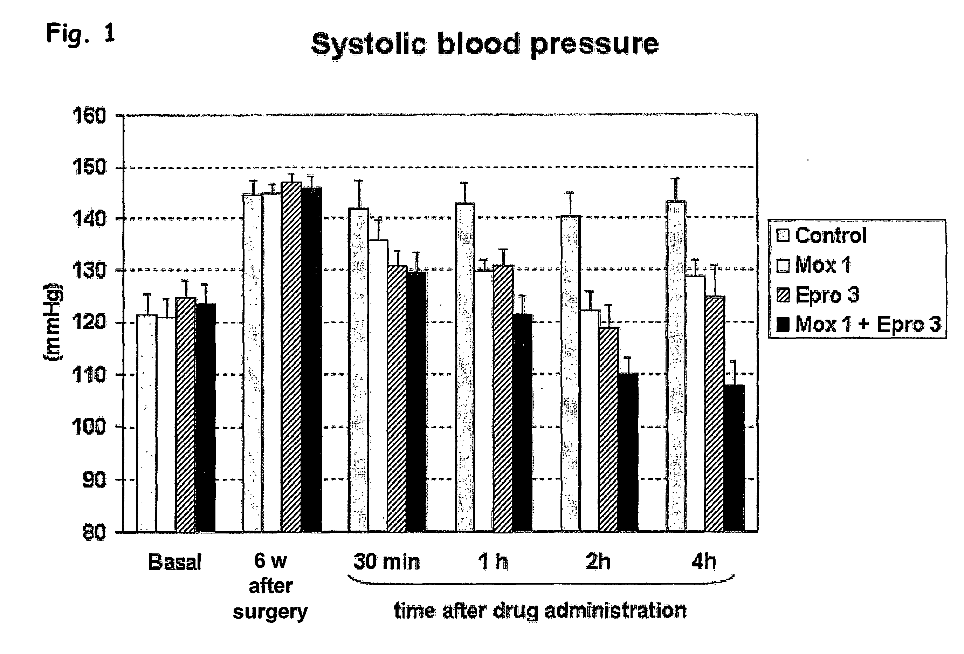

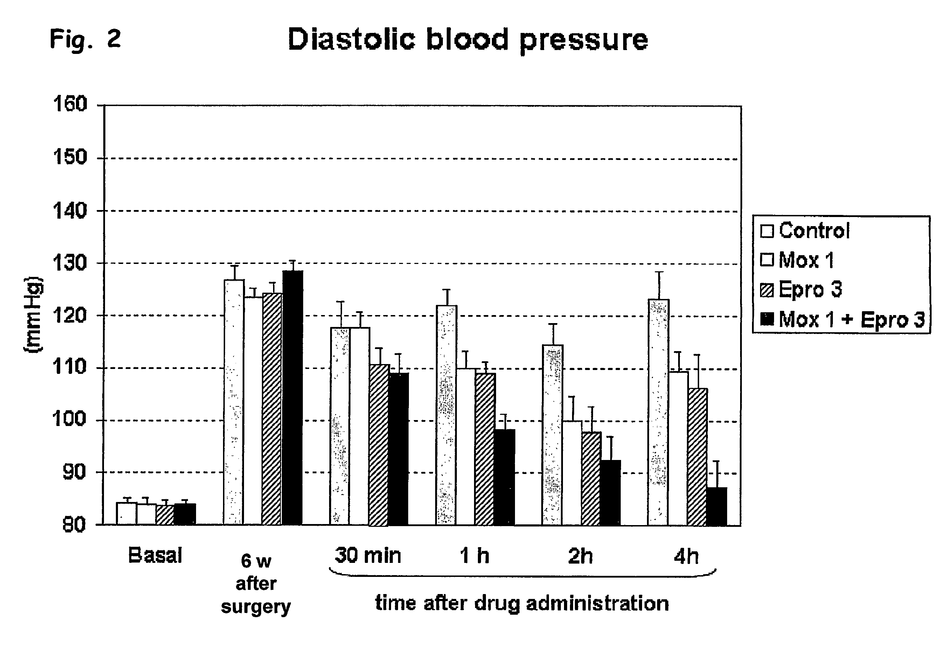

The effects of moxonidine (1 mg/kg), eprosartan (3 mg/kg) and their combination on the blood pressure are demonstrated in Table 1 and Figures 1 and 2.

Moxonidine treatment caused a significant decrease in blood pressure of renal hypertensive rats, showing a maximal effect 2 hours after the administration of the drug (Table 1; 16 % and 19 % decrease in the systolic and diastolic blood pressure, respectively). There was a moderate recovery of blood pressure 4 hours after treatment.

Administration of eprosartan also significantly decreased the blood pressure (Table 1), with maximal effects of 19 % and 21 % (systolic and diastolic blood pressure, respectively) 2 hours after the treatment.

Administration of the two drugs together resulted in a significantly stronger decrease in both the systolic and diastolic blood pressure compared to the blood pressure effects of Moxonidine and Eprosartan alone (Table 1, Figures 1 and 2), reaching maximum values 4 hours after administration (26 % and 33 % decrease in systolic and diastolic blood pressure, respectively).

Heart rate did not change significantly during the experiment in the vehicle treated animals. None of the treatments induced significant alterations in the heart rate during the investigations (Table 2).

**Table 1:**

| Effect of oral treatment with moxonidine (Mox), eprosartan (Epro) or their combination on the blood pressure of renal hypertensive rats 6 weeks after narrowing of the right renal artery ('two kidney - one clip hypertension'). | | | | | | | | |
|---|---|---|---|---|---|---|---|---|
| **Group** | **BW (g)** | **Blood Pressure** | **Before surgery** | **After surgery** | | | | |
| | | | | **6^{th} week** | **After drug administration** | | | |
| | | | | | **30 min** | **1 h** | **2 h** | **4 h** |
| **Control** | 351±8.6 | **SBP** | 121±4.0 | 145±2.7 | 142±5.4 | 143±4.2 | 140±4.5 | 143±4.7 |
| | | **MBP** | 97±1.4 | 133±2.4 | 126±5.2 | 129±3.3 | 123±3.9 | 130±4.9 |
| | | **DBP** | 84±1.0 | 127+2.5 | 118±5.0 | 122±2.9 | 114±4.2 | 123±5.1 |
| **Mox 1 mg/kg** | 346±4.4 | **SBP** | 121±3.7 | 145±1.8 | 136±3.6 | 130±2.1* | 122±3.5* | 129±3.1 |
| | | **MBP** | 96±1.0 | 130±1.3 | 124±3.2 | 117±2.6* | 107±4.2* | 116±3.5 |
| | | **DBP** | 84±1.2 | 124±1.6 | 118±3.1 | 110±3.3* | 100±4.8 | 109±3.8 |
| **Epro 3mg/kg** | 347±8.6 | **SBP** | 125±3.3 | 147±1.9 | 131±2.8 | 131±3.1* | 119±4.5* | 125±6.1* |
| | | **MBP** | 98±1.0 | 131±1.6 | 117±2.8 | 116±2.5 * | 105±4.6 * | 112±6.6* |
| | | **DBP** | 84±1.0 | 124±2.0 | 111±2.8 | 109±2.3 * | 98±4.8 * | 106±6.6 * |
| **Mox 1 mg/kg + Epro 3mg/kg** | 3455.3 | **SBP** | 124±3.6 | 146±2.1 | 130±3.8 | 122±3.5 * | 110±3.1* | 108±4.7 *§# |
| | | **MBP** | 97±1.3 | 134±1.9 | 116±3.8 | 106±2.8 *§# | 98±3.6 * | 94±4.8 *§# |
| | | **DBP** | 84±0.9 | 129±2.0 | 109±3.9 | 98±3.1*§# | 92±4.4* | 87±4.9*§# |

| | | | | | | | | |
|---|---|---|---|---|---|---|---|---|
| BW = body weight of the animals; SBP = systolic blood pressure (mmHg); MBP = mean blood pressure (mmHg); DBP = diastolic blood pressure (mmHg). Results are mean ± SE of 12 animals. Asterisks denote statistically significant difference (P<0.05) compared to the * control (vehicle treated), § moxonidine or # eprosartan treated animals. | | | | | | | | |

**Table 2:**

| Effect of oral treatment with moxonidine (Mox), eprosartan (Epro) or their combination on the heart rate (beats/min) of renal hypertensive rats 6 weeks after narrowing of the right renal artery ('two kidney - one clip hypertension'). (For details see table 1) | | | | | | | |
|---|---|---|---|---|---|---|---|
| **Group** | **n** | **Before surgery** | **After surgery** | | | | |
| | | | **6th week** | **After drug administration** | | | |
| | | | | **30 min** | **1 h** | **2 h** | **4 h** |
| **Control** | 12 | 378±6.2 | 358±7.1 | 361±5.3 | 361±6.8 | 367±5.4 | 374±4.7 |
| **Mox 1mg/kg** | 12 | 386±8.1 | 371±7.6 | 383±5.3 | 376±6.7 | 368±5.9 | 384±6.0 |
| **Epro 3mg/kg** | 12 | 385±7.7 | 373±6.4 | 370±6.9 | 375±7.4 | 378±4.3 | 376±5.2 |
| **Mox 1mg/kg+ Epro 3mg/kg** | 12 | 368±7.0 | 361±7.4 | 374±5.3 | 363±12.4 | 374±4.6 | 377±6.3 |

### 4. Conclusion

Both moxonidine and eprosartan significantly decreased the blood pressure of renal hypertensive (2K1C) rats. The combination of the two drugs shows significant synergistic effects. The combination produces surprisingly strong blood pressure lowering effects compared to the single compounds. The marked antihypertensive effect of the combination does suggest that it is reasonable to use smaller doses of the individual compounds to achieve the same antihypertensive effect with less adverse side effects.

These results were confirmed by an independently later published clinical study on 10 human patients suffering from hypertensive chronic renal failure [Neumann J et al. (2003) "Eprosartan combined with moxonidine normalizes sympathetic hyperactivity in hypertensive chronic renal failure patients" J Am Soc Nephrol; 14:20A]. It was shown that the combined treatment of Eprosartan with Moxonidine reduced sympathetic activity to normal levels.

### EXAMPLE 3 - PHARMACOLOGICAL ASSAY FOR GLUCOSE TOLERANCE

### 1. Introduction

The effect of a combined administration of Moxonidine as an example for a selective Imidazoline I1 receptor agonist and Eprosartan as an example for an Angiotensin II At1 receptor antagonist was analyzed by measuring their influence on plasma glucose level in "Zucker rats". The "Zucker rat" is a model of impaired glucose tolerance and is widely used to analyze compound effects on glucose tolerance.

### 2. Methods

Animals: Male Zucker rats (HsdOla fa/fa) from Harlan were used in the experiments. The animals were fed commercial lab chow and had unlimited access to tap water throughout the experiment.

Experimental protocol: Animals were treated for 3 weeks with either vehicle or the active compounds. Moxonidine was applied via the drinking water. Eprosartan was administered daily into the stomach via a cannula. Ten animals were used in each of the following treatment groups:
- Vehicle
- Moxonidine 1 mg/kg
- Moxonidine 1 mg/kg + Eprosartan 3 mg/kg
- Moxonidine 1 mg/kg + Eprosartan 30 mg/kg
- Moxonidine 1 mg/kg + Eprosartan 100 mg/kg

At the end of the drug treatment period the animals were subjected to an oral glucose tolerance test. Animals were given a solution containing 2 g glucose via a cannula into the stomach. Blood samples were drawn before and at 30, 60, 90, and 120 minutes after the glucose load by tail vein cannulation and analyzed for plasma glucose levels.

Statistical analysis: Parameters were expressed as mean ± standard error of the mean (SE) and after analysis of variance were compared by means of T- Test.

### 3. Results

The effects of Moxonidine (1 mg/kg) alone and in combination with increasing doses (3 mg/kg; 30 mg/kg; 100 mg/kg) of Eprosartan are presented in Table 3.

Moxonidine treatment resulted in a significant reduction in plasma glucose levels at 60 and 90 minutes after the oral glucose challenge.

Combined administration of Moxonidine (1 mg/kg) and Eprosartan caused a further dose-dependent reduction in plasma glucose level. This further reduction was significant at 90 and 120 minutes in the group dosed with 30 mg Eprosartan and at 30, 90, and 120 minutes in the group receiving 100 mg/kg Eprosartan in addition to Moxonidine.

### 4. Conclusion

The above presented experiments showed that even in view of the well-known anti-hyperglycemic effects of Moxondine, the combination of Moxonidine with Eprosartan showed further dose-dependent reduction of the plasma glucose levels, thereby showing synergistic effects. The drug combination has surprisingly strong plasma glucose lowering effects in a model for impaired glucose tolerance compared to the single compounds. This additional characteristic next to the marked antihypertensive effect of the drug combination makes it ideally suited for the treatment of hypertensive patients suffering from metabolic impairment, i.e. insulin resistance, hyperglycemia, and/or diabetes mellitus.

**Table 3:**

| Effect of oral treatment with moxonidine (Mox) or the combined treatment with moxonidine (Mox) and eprosartan (Epro) on the plasma glucose levels of Zucker rats after 30, 60, 90 or 120 min after glucose challenge. | | | | | | | |
|---|---|---|---|---|---|---|---|
| **Group** | **N** | **Minutes after glucose challenge** | | | | | **AUC** **(mg/dl*min)** |
| | | **0** | **30** | **60** | **90** | **120** | |
| **Control** | 10 | 93 ± 4 | 269 ± 8 | 260 ± 17 | 288 ± 26 | 283 ± 26 | 30161±1712 |
| **1 mg/kg Mox** | 10 | 97 ± 6 | 240 ± 15 | 211 ± 15 * | 221 ± 11 * | 224 ± 14 | 24960±1268 * |
| **1 mg/kg Mox + 3 mg/kg Epro** | 10 | 75 ± 3 *§ | 226 ± 25 | 192 ± 17 * | 224 ± 21 | 222 ± 17 | 23729±2097 * |
| **1 mg/kg Mox + 30 mg/kg Epro** | 10 | 90 ± 12 | 225 ± 9 * | 205 ± 13 * | 182 ± 11 *§ | 181 ± 12 *§ | 22415±911 * |
| **1 mg/kg Mox + 100 mg/kg Epro** | 10 | 87 ± 3 | 159 ± 12 *§ | 177 ± 10 * | 181 ± 14 *§ | 156 ± 10 *§ | 19172±1160 *§ |

| | | | | | | | |
|---|---|---|---|---|---|---|---|
| Glucose levels are given in mg/dl. The data represent means ± SE of 10 animals. AUC = "area under the curve" Arterisks denote statistically significant differences (p<0.05) compared to control * vehicle-treated or § Moxonidine-treated animals (T-test). | | | | | | | |

From the aforementioned pharmacological tests it becomes clear, that the combination of a selective imidazoline receptor agonist such as Moxonidine together with an angiotensin II receptor blocker such as Eprosartan provides a potential novel treatment method for hypertensive patients in need of a stringent control of their blood pressure levels to below previously existing target levels.

Furthermore, there is evidence that the use of ARBs may prevent the occurrence of diabetes in hypertensive individuals and will reduce cardiovascular events in diabetics. ARBs have been shown to slow the progression of renal disease in diabetic patients and prevent the occurrence of end-stage renal disease when compared to treatment regimens that do not include an ARB. On the other hand, Moxonidine is known to beneficially influence metabolism in diabetic patients. Moxonidine is able to reduce plasma insulin already in patients with impaired glucose tolerance where fasting plasma glucose is not yet influenced.

Furthermore, a complementary pharmacological activity of the combination Moxonidine/Eprosartan is expected particularly in low renin hypertension and in systolic hypertension. The low response rate of Eprosartan in spontaneously hypertensive rats (SHR), a model for low renin hypertension, will be increased by the combination with Moxonidine which shows excellent activity in SHR.

ARBs are described to inhibit catecholamine outflow from sympathetic nerve terminals by blockade of presynaptic AT1 receptors. This effect was especially described for Eprosartan [Ohlstein EH et al. (1997) Pharmacology 55:244-251]. This pronounced peripheral sympatholytic activity of Eprosartan coupled with the central sympatholytic properties of Moxonidine appears to reveal additive and/or synergistic effects particularly in systolic hypertension and heart failure.

Furthermore, hypertension is often associated with metabolic impairment (insulin resistance, hyperglycemia, diabetes mellitus type II, and/or hyperlipidemia) and both are linked by excessive activity of the sympathetic nervous system. Thus, in a further aspect of the present invention, the dual peripheral/central sympatholytic effects of the combination Moxonidine/Eprosartan represent an ideal drug combination for the treatment of hypertension associated with insulin resistance, hyperglycemia and/or diabetes mellitus. This was proven by the combined administration of Moxonidine with Eprosartan which showed an additional synergistic effect on the plasma glucose levels.

Stimulation of both, the SNS and RAAS reveals neurohormonal activation (increase of catecholamines, renin, angiotensin II and aldosterone plasma levels) and consequently promotes structural remodelling of vascular, cardiac and renal tissue as seen in chronic heart failure and renal disease. Therefore optimal drug treatment for end organ disease would attenuate both the SNS and RAAS. Moxonidine, through activation of Imidazoline I1 receptors in both the brain stem and the kidneys, at sub-antihypertensive doses attenuates sympathetic over-activity and consequently ameliorates glomerulosclerosis, proteinuria and renal remodelling in rats whereas Angiotensin receptor blockers like Eprosartan are known to reduce cardiac, renal and vascular structural and functional damage due to blockade of the AT1 receptor.

In conclusion, the present invention provides with the combined administration of Moxonidine and Eprosartan within one pharmaceutical preparation a novel highly effective treatment method of hypertension, particularly systolic hypertension and hypertension associated with metabolic and renal impairment and heart failure, because this combination inhibits the two main pressure systems SNS and RAAS and consequently neurohormonal activation. This profile of action fulfills the requirements set up for an ideal combination therapy, i.e. complementary mechanism of action and enhanced efficacy. The excellent tolerability of both Moxonidine and Eprosartan is expected to be maintained given that both drugs possess excellent safety profiles.

### CITED LITERATURE

1. Aranda P. et al. (1999) "Irbesartan plus Moxonidine: a logical combination for hypertensive patients not responding to monotherapy" (Conference abstract: 13th Scientific Meeting of the Inter-American Society of Hypertension, USA) Hypertension. 33(4):1065
2. Bock et al. (1999) Analysis of the receptor involved in the central hypotensive effect of rilmenidine and moxonidine. Naunyn Schmiedebergs Arch Pharmacol. 359:262-71
3. Chobanian AV et al. (2003) "The Seventh Report of the Joint National Committee on Prevention, Detection, Evaluation, and Treatment of High Blood Pressure: the JNC 7 report." JAMA. 289(19):2560-72
4. EP 0253310
5. EP 0403159B1
6. EP 0443983B1
7. EP 0454511B1
8. EP 0689837
9. Ernsberger (2000) Pharmacology of moxonidine: an I1-imidazoline receptor agonist. J Cardiovasc Pharmacol. 35:S27-41
10. European Society of Hypertension-European Society of Cardiology Guidelines Committee (2003) "2003 European Society of Hypertension-European Society of Cardiology guidelines for the management of arterial hypertension" J Hypertens. 21 (6):1011-53
11. Farsang C. (2001) "Moxonidine: Clinical profile" J Clin Basic Cardiol 4:197-200
12. Hansson L. et al. (1998) "Effects of intensive blood-pressure lowering and low-dose aspirin in patients with hypertension: principal results of the Hypertension Optimal Treatment (HOT) randomised trial. HOT Study Group" Lancet 351 (9118):1755-62
13. Le Bihan et al. (1999) Design and synthesis of imidazoline derivatives active on glucose homeostasis in a rat model of type II diabetes. 2. Syntheses and biological activities of 1,4-dialkyl-, 1,4-dibenzyl, and 1-benzyl-4-alkyl-2-(4',5'-dihydro-1'H-imidazol-2'-yl)piperazines and isosteric analogues of imidazoline. J Med Chem. 42:1587-603
14. Neumann J et al. (2003) "Eprosartan combined with moxonidine normalizes sympathetic hyperactivity in hypertensive chronic renal failure patients" J Am Soc Nephrol; 14:20A
15. Ohlstein EH et al. (1997) "Inhibition of sympathetic outflow by the angiotensin II receptor antagonist, Eprosartan, but not by losartan, valsartan or irbesartan: relationship to differences in prejunctional angiotensin II receptor blockade." Pharmacology 55:244-251
16. Prichard et al. (2002) "Placebo-controlled comparison of the efficacy and tolerability of once-daily Moxonidine and enalapril in mild to moderate essential hypertension". Blood Press 11 (3):166-72
17. Rondu et al. (1997) Design and synthesis of imidazoline derivatives active on glucose homeostasis in a rat model of type II diabetes. 1. Synthesis and biological activities of N-benzyl-N'-(arylalkyl)-2-(4',5'-dihydro-1'H-imidazol-2'-yl)piperazines. J Med Chem. 40:3793-803
18. Rupp H and Jäger B. (2001) "The renin-angiotensin system and the sympathetic nervous system in hypertension and congestive heart failure: Implications for therapeutic interventions." J Clin Basic Cardiol 4:47-51
19. Schann et al. (2001) Synthesis and biological evaluation of pyrrolinic isosteres of rilmenidine. Discovery of cis-/trans-dicyclopropylmethyl-(4,5-dimethyl-4,5-dihydro-3H-pyrrol-2-yl)-amine (LNP 509), an I1 imidazoline receptor selective ligand with hypotensive activity. J Med Chem. 44):1588-93
20. Shusterman N. (2002) " Risk-benefit assessment of angiotensin II receptor antagonists." Expert Opin Drug Saf. 1(2):137-52
21. Taylor Nelson Sofres Healthcare, Cardio Monitor® Study (1998)
22. Trenkwalder P. (2002) "Combination therapy with AT1-receptor blockers." J of Human Hypertension 16, Suppl 3: S17- S25
23. US 5,185,351
24. US 5,270,317
25. US 5,399,578
26. US 5,409,947
27. US 6,235,311
28. Vetter H and Düsing R. (1997) "Moderne Basis- und Kombinationstherapie der Hypertonie." Nieren- und Hochdruckkrankheiten 26(31):105-107
29. Wallenstein S et al. (1980) "Some statistical methods useful in circulation research" Circ. Res. 47:1-9
30. Waters J. et al. (1999) "Use of Moxonidine as initial therapy and in combination in the treatment of essential hypertension - results of the TOPIC (Trial Of Physiotens In Combination) study" J Clin Bas Cardiol. 2(2):219-24
31. WO 00/02878
32. WO 01/41764
33. Zanchetti A & Ruilope LM (2002) "Antihypertensive treatment in patients with type-2 diabetes mellitus: what guidance from recent controlled randomized trials" J Hypertension; 20:2099- 2110

## Claims

1. A pharmaceutical composition comprising moxonidine or a pharmaceutically acceptable salt thereof as selective I1 imidazoline receptor agonist and eprosartan or a pharmaceutically acceptable salt thereof as angiotensin II receptor blocker and a pharmaceutically acceptable carrier.

2. The pharmaceutical composition according to claim 1 wherein the composition consists of a fixed combination of moxonidine or a pharmaceutically acceptable salt thereof and eprosartan mesylate and a pharmaceutically acceptable carrier.

3. The pharmaceutical composition according to claim 2, wherein the Moxonidine is present in a dose of 0.05 - 1 mg, preferably 0.2 - 0.6 mg, and the Eprosartan is present in a dose of 100 - 1000 mg, preferably 300 - 600 mg..

4. The pharmaceutical composition according to claim 2 or 3, wherein the Moxonidine is present in a dose of 0.2 mg or 0.4 mg and the Eprosartan is present in a dose of 600 mg.

5. The pharmaceutical composition according to any of the claims 2 to 4, wherein the pharmaceutical composition is in the form of a tablet consisting mainly of Eprosartan and further of Moxonidine homogenously distributed within the Eprosartan.

6. The pharmaceutical composition according to any of the claims 2 to 4, wherein the pharmaceutical composition is in the form of a coated tablet wherein a small Moxonidine containing core is coated with an Eprosartan containing blend.

7. The pharmaceutical composition according to any of the claims 2 to 4, wherein the pharmaceutical composition is in the form of an Eprosartan containing tablet core coated with a thin layer comprising the Moxonidine.

8. The pharmaceutical composition according to any of the claims 2 to 4, wherein the pharmaceutical composition is in the form of a bilayer tablet.

9. The pharmaceutical composition according to any of the claims 2 to 4, wherein the pharmaceutical composition is in the form of a trilayer tablet.

10. The pharmaceutical composition according to any of the preceding claims wherein the composition additionally comprises a diuretic, in particular hydrochlorothiazide.

11. Use of a therapeutically effective amount of Moxonidine as selective I1 imidazoline receptor agonist and of a therapeutically effective amount of Eprosartan as angiotensin II receptor blocker for the manufacture of a medicament for the treatment of a subject suffering from or being susceptible to hypertension, in particular systolic hypertension.

12. Use according to claim 11, wherein the Eprosartan is present in an amount in a daily dosage range from 100 - 1000 mg, preferably 300 - 600 mg, and wherein the Moxonidine is present in an amount in a daily dosage range from 0.05 - 1 mg, preferably from 0.2 - 0.6 mg.

13. Use according to claim 12, wherein the Eprosartan is present in an amount in a daily dosage of 600 mg, and the Moxonidine is present in an amount in a daily dosage of 0.2 mg or 0.4 mg.

14. Use according to any of the claims 11 to 13, wherein the subject suffers from or is susceptible to hypertension, in particular systolic hypertension, associated with metabolic impairment.

15. Use according to claim 14, wherein the metabolic impairment is **characterized by** insulin resistance, hyperglycemia and/or hyperlipidemia.

16. Use according to any of the claims 11 to 15, wherein the subject is suffering from or being susceptible to hypertension, in particular systolic hypertension, associated with diabetes mellitus type II.

17. Use according to any of the claims 11 to 16, wherein the hypertension, in particular systolic hypertension, is further associated with renal impairment.

18. Use according to any of the claims 11 to 17, wherein the hypertension, in particular systolic hypertension, is further associated with heart failure.

19. Use according to any of the preceding claim 11 to 18, wherein the medicament further comprises a therapeutically effective amount of a diuretic, in particular hydrochlorothiazide.

## Patentansprüche

1. Pharmazeutische Zusammensetzung, umfassend Moxonidin oder ein pharmazeutisch annehmbares Salz hiervon als selektiven I1 Imidazolin-Rezeptor-Agonisten und Eprosartan oder ein pharmazeutisch annehmbares Salz hievon als Angiotensin-II-Rezeptor-Blocker und einen pharmazeutisch annehmbaren Träger.

2. Pharmazeutische Zusammensetzung nach Anspruch 1, wobei die Zusammensetzung aus einer festgelegten Kombination von Moxonidin oder einem pharmazeutisch annehmbaren Salz hiervon und Eprosartanmesylat und einem pharmazeutisch annehmbaren Träger besteht.

3. Pharmazeutische Zusammensetzung nach Anspruch 2, worin Moxonidin in einer Dosis von 0,05 - 1 mg, vorzugsweise von 0,2 - 0,6 mg, vorliegt und Eprosartan in einer Dosis von 100 - 1.000 mg, vorzugsweise von 300 - 600 mg, vorliegt.

4. Pharmazeutische Zusammensetzung nach Anspruch 2 oder 3, worin Moxonidin in einer Dosis von 0,2 mg oder 0,4 mg vorliegt und Eprosartan in einer Dosis von 600 mg vorliegt.

5. Pharmazeutische Zusammensetzung nach einem der Ansprüche 2 bis 4, wobei die pharmazeutische Zusammensetzung in Form einer Tablette vorliegt, welche überwiegend aus Eprosartan besteht und ferner Moxonidin in Eprosartan homogen verteilt enthält.

6. Pharmazeutische Zusammensetzung nach einem der Ansprüche 2 bis 4, wobei die pharmazeutische Zusammensetzung in Form einer beschichteten Tablette vorliegt, worin ein kleiner Moxonidin enthaltender Kern mit einer Eprosartan enthaltenden Mischung beschichtet ist.

7. Pharmazeutische Zusammensetzung nach einem der Ansprüche 2 bis 4, wobei die pharmazeutische Zusammensetzung in Form eines Eprosartan enthaltenden Tablettenkerns vorliegt, welcher mit einer Moxonidin umfassenden dünnen Schicht beschichtet ist.

8. Pharmazeutische Zusammensetzung nach einem der Ansprüche 2 bis 4, wobei die pharmazeutische Zusammensetzung in Form einer Zweischichttablette vorliegt.

9. Pharmazeutische Zusammensetzung nach einem der Ansprüche 2 bis 4, wobei die pharmazeutische Zusammensetzung in Form einer Dreischichttablette vorliegt.

10. Pharmazeutische Zusammensetzung nach einem der vorstehenden Ansprüche, wobei die Zusammensetzung zusätzlich ein Diuretikum, insbesondere Hydrochlorthiazid, umfasst.

11. Verwendung einer therapeutisch wirksamen Menge von Moxonidin als selektiven I1 Imidazolin-Rezeptor-Agonisten und einer therapeutisch wirksamen Menge von Eprosartan als Angiotensin-II-Rezeptor-Blocker zur Herstellung eines Arzneimittels zur Behandlung eines Patienten, welcher unter Bluthochdruck, insbesondere systolischem Bluthochdruck, leidet oder dafür anfällig ist.

12. Verwendung nach Anspruch 11, wobei Eprosartan in einer Menge im täglichen Dosierungsbereich von 100 - 1.000 mg, vorzugsweise von 300 - 600 mg, vorliegt und worin Moxonidin in einer Menge im täglichen Dosierungsbereich von 0,05 - 1 mg, vorzugsweise von 0,2 - 0,6 mg, vorliegt.

13. Verwendung nach Anspruch 12, wobei Eprosartan in einer Menge in einer täglichen Dosierung von 600 mg vorliegt und Moxonidin in einer Menge in einer täglichen Dosierung von 0,2 mg oder 0,4 mg vorliegt.

14. Verwendung nach einem der Ansprüche 11 bis 13, wobei der Patient unter Bluthochdruck, insbesondere systolischem Bluthochdruck, in Verbindung mit metabolischer Beeinträchtigung leidet oder dafür anfällig ist.

15. Verwendung nach Anspruch 14, wobei die metabolische Beeinträchtigung durch Insulinresistenz, Hyperglykämie und/oder Hyperlipidämie **gekennzeichnet** ist.

16. Verwendung nach einem der Ansprüche 11 bis 15, wobei der Patient unter Bluthochdruck, insbesondere systolischem Bluthochdruck, in Verbindung mit Diabetes mellitus Typ II leidet oder dafür anfällig ist.

17. Verwendung nach einem der Ansprüche 11 bis 16, wobei der Bluthochdruck, insbesondere der systolische Bluthochdruck, ferner mit einer Nierenbeeinträchtigung verbunden ist.

18. Verwendung nach einem der Ansprüche 11 bis 17, wobei der Bluthochdruck, insbesondere der systolische Bluthochdruck, ferner mit Herzinsuffizienz verbunden ist.

19. Verwendung nach einem der vorstehenden Ansprüche 11 bis 18, wobei das Arzneimittel ferner eine therapeutisch wirksame Menge eines Diuretikums, insbesondere von Hydrochlorthiazid, umfasst.

## Revendications

1. Composition pharmaceutique comprenant de la moxonidine ou un sel pharmaceutiquement acceptable de celle-ci en tant qu'agoniste des récepteurs de l'imidazoline I1 sélectif et de l'éprosartan ou un sel pharmaceutiquement acceptable de celui-ci en tant que bloqueur des récepteurs de l'angiotensine II et un support pharmaceutiquement acceptable.

2. Composition pharmaceutique selon la revendication 1, dans laquelle la composition consiste en une combinaison fixe de moxonidine ou d'un sel pharmaceutiquement acceptable de celle-ci et de mésylate d'éprosartan et un support pharmaceutiquement acceptable.

3. Composition pharmaceutique selon la revendication 2, dans laquelle la moxonidine est présente à une dose de 0,05 à 1 mg, de préférence de 0,2 à 0,6 mg, et l'éprosartan est présent à une dose de 100 à 1 000 mg, de préférence de 300 à 600 mg.

4. Composition pharmaceutique selon la revendication 2 ou 3, dans laquelle la moxonidine est présente à une dose de 0,2 mg ou 0,4 mg et l'éprosartan est présent à une dose de 600 mg.

5. Composition pharmaceutique selon l'une quelconque des revendications 2 à 4, dans laquelle la composition pharmaceutique se présente sous la forme d'un comprimé consistant principalement en de l'éprosartan et en outre de la moxonidine distribuée de manière homogène dans l'éprosartan.

6. Composition pharmaceutique selon l'une quelconque des revendications 2 à 4, dans laquelle la composition pharmaceutique se présente sous la forme d'un comprimé enrobé dans lequel un petit noyau contenant de la moxonidine est enrobé avec un mélange contenant de l'éprosartan.

7. Composition pharmaceutique selon l'une quelconque des revendications 2 à 4, dans laquelle la composition pharmaceutique se présente sous la forme d'un noyau de comprimé contenant de l'éprosartan enrobé d'une fine couche comprenant la moxonidine.

8. Composition pharmaceutique selon l'une quelconque des revendications 2 à 4, dans laquelle la composition pharmaceutique se présente sous la forme d'un comprimé bicouche.

9. Composition pharmaceutique selon l'une quelconque des revendications 2 à 4, dans laquelle la composition pharmaceutique se présente sous la forme d'un comprimé tricouche.

10. Composition pharmaceutique selon l'une quelconque des revendications précédentes, dans laquelle la composition comprend en outre un diurétique, en particulier l'hydrochlorothiazide.

11. Utilisation d'une quantité thérapeutiquement efficace de moxonidine en tant qu'agoniste des récepteurs de l'imidazoline I1 sélectif et d'une quantité thérapeutiquement efficace d'éprosartan en tant que bloqueur des récepteurs de l'angiotensine II pour la fabrication d'un médicament destiné au traitement d'un sujet souffrant de ou étant prédisposé à une hypertension, en particulier une hypertension systolique.

12. Utilisation selon la revendication 11, dans laquelle l'éprosartan est présent en une quantité dans une gamme posologique quotidienne de 100 à 1 000 mg, de préférence de 300 à 600 mg et dans laquelle la moxonidine est présente en une quantité dans une gamme posologique quotidienne de 0,05 à 1 mg, de préférence de 0,2 à 0,6 mg.

13. Utilisation selon la revendication 12, dans laquelle l'éprosartan est présent en une quantité dans une posologie quotidienne de 600 mg, et la moxonidine est présente en une quantité dans une posologie quotidienne de 0,2 mg ou 0,4 mg.

14. Utilisation selon l'une quelconque des revendications 11 à 13, dans laquelle le sujet souffre de ou est prédisposé à une hypertension, en particulier une hypertension systolique, associée à une déficience métabolique.

15. Utilisation selon la revendication 14, dans laquelle la déficience métabolique est **caractérisée par** une résistance à l'insuline, une hyperglycémie et/ou une hyperlipidémie.

16. Utilisation selon l'une quelconque des revendications 11 à 15, dans laquelle le sujet souffre de ou est prédisposé à une hypertension, en particulier une hypertension systolique, associée à un diabète sucré de type II.

17. Utilisation selon l'une quelconque des revendications 11 à 16, dans laquelle l'hypertension, en particulier l'hypertension systolique, est en outre associée à une déficience rénale.

18. Utilisation selon l'une quelconque des revendications 11 à 17, dans laquelle l'hypertension, en particulier l'hypertension systolique, est en outre associée à une insuffisance cardiaque.

19. Utilisation selon l'une quelconque des revendications 11 à 18 précédentes, dans laquelle le médicament comprend en outre une quantité thérapeutiquement efficace d'un diurétique, en particulier l'hydrochlorothiazide.
